# FASCICULE DE BREVET EUROPEEN

(11) **EP 0 831 972 B1**
(45) Date de publication et mention de la délivrance du brevet: **24.11.1999**
(21) Numéro de dépôt: 96924939.0
(22) Date de dépôt: 05.07.1996
(51) Int. Cl.: B05B 11/00, B65D 47/34

(54) **DISPOSITIF DE DISTRIBUTION DE PRODUIT FLUIDE A ACTION BACTERIOSTATIQUE**
VORRICHTUNG MIT BAKTERIOSTATISCHER WIRKUNG ZUR ABGABE VON FLIESSFÄHIGEN PRODUKTEN
DEVICE FOR DISPENSING A FLUID HAVING BACTERIOSTATIC ACTIVITY

(30) Priorité: 07.07.1995 FR 9508263
(43) Date de publication de la demande: 01.04.1998
(73) Titulaire: VALOIS S.A., 27110 Le Neubourg (FR)
(72) Inventeur: GARCIA, Firmin, F-27000 Evreux (FR); FOURMENT, Olivier, F-75016 Paris (FR); BROUET, Guillaume, Connecticut 06490 (US); ARGHYRIS, Laurent, F-76300 Sotteville-lès-Rouen (FR)
(74) Mandataire: CAPRI SARL
(86) Numéro de dépôt international: FR9601048
(87) Numéro de publication internationale: WO9702900

(56) Documents cités:
- EP-A- 0 473 892
- EP-A- 0 534 088
- EP-A- 0 580 460
- DE-A- 2 830 977
- US-A- 4 801 093

## Description

La présente invention concerne un dispositif de distribution de produit fluide à action bactériostatique.

Il est connu d'utiliser des métaux lourds, tel que l'argent, des alliages de ceux-ci, ou des sels de ceux-ci en tant que substance à action oligodynamique ou bactériostatique, c est-à-dire anti-bactérienne pour des produits fluides. En effet, l'argent est soluble dans l'eau dans des concentrations ppb, et les ions d'argent ainsi délivrés agissent de manière bactériostatique et bactéricide sur les germes pénétrant dans le produit, pour les éliminer.

Le document EP-0 473 892 divulgue une pompe-doseuse sans reprise d'air ayant une substance oligodynamique disposée au niveau du clapet d'admission de la chambre de pompe. De manière additionnelle, la substance oligodynamique peut aussi être disposée dans d'autres parties de la pompe-doseuse. Le but de ce dispositif, tel que décrit dans le document EP-0 473 892, est de garantir une élimination des germes qui soit particulièrement efficace. Ce but est obtenu en fournissant un contact prolongé du liquide avec la substance oligodynamique. En effet, de par la présence de cette substance au niveau du clapet d'entrée de la chambre de pompe, aussi bien une partie du produit contenu dans le réservoir que le produit contenu dans la chambre de pompe est en contact permanent avec la substance oligodynamique. Ceci est encore amplifié si d'autres parties de la pompe, tel que la chambre de pompe, elle-même, ou le clapet de sortie, comportent la substance oligodynamique.

Cette mise en oeuvre présente un certain nombre d'inconvénients. Ainsi, selon la nature du produit contenu dans le récipient, le contact permanent entre une partie dudit produit et la substance oligodynamique peut entraîner des problèmes de stabilité dudit produit pendant le stockage. D'autre part, la fabrication d'une pompe-doseuse est plus compliquée et donc plus coûteuse si une ou plusieurs parties constitutives de ladite pompe doivent comporter une substance oligodynamique.

Le document EP-0 580 460 divulgue un distributeur de produit fluide dans lequel le produit bactériostatique peut n'être prévu que dans le poussoir. Toutefois, dans ce cas, l'efficacité de l'action bactériostatique peut être limitée, et selon la configuration des parties constitutives du poussoir, une partie du produit expulsé lors de l'actionnement risque de ne pas entrer en contact avec la substance oligodynamique. De même, si le temps de stockage est relativement court, c'est-à-dire si le distributeur est réutilisé rapidement, une partie du produit restant dans le poussoir entre deux actionnements successifs risque de se contaminer. D'autre part, si le temps de stockage entre deux actionnements est long, une quantité non négligeable de produit est en contact permanent avec la substance bactériostatique, ce qui n'est pas souhaitable.

La présente invention a donc pour but de fournir un dispositif de distribution de produit fluide ayant une action bactériostatique efficace et ne présentant pas de risque d'instabilité pour ledit produit fluide.

La présente invention a aussi pour but de fournir un dispositif de distribution de produit fluide ayant une action bactériostatique efficace, dans lequel la totalité ou la quasitotalité du produit fluide n'est pas en contact permanent avec la substance bactériostatique.

La présente invention a encore pour but de fournir un dispositif de distribution de produit fluide ayant une action bactériostatique efficace, qui soit simple et peu coûteux à réaliser.

La présente invention a donc pour objet un dispositif de distribution de produit fluide fonctionnant sans reprise d'air comportant une pompe montée sur un récipient contenant le produit, et un poussoir pour actionner ladite pompe, ledit poussoir comprenant un orifice de distribution de produit relié à la pompe par un canal d'expulsion, une substance ayant une action bactériostatique sur ledit produit lorsque celui-ci vient en contact avec ladite substance étant disposée dans ledit dispositif de distribution, caractérisé en ce que ledit poussoir comporte un gicleur réalisé sous la forme d'un insert mis en place dans le canal d'expulsion et remplissant sensiblement tout le volume disponible entre la pompe et l'orifice de distribution de manière à limiter au maximum le volume mort, ladite substance à action bactériostatique étant disposée uniquement dans ledit poussoir, sur ou à proximité dudit gicleur, de sorte que ladite substance à action bactériostatique a une efficacité maximale en raison du faible volume mort dans le poussoir.

En particulier ledit poussoir est un poussoir nasal comportant un gicleur interne remplissant sensiblement tout le volume mort et définissant un étroit canal d'expulsion, ladite substance à action bactériostatique étant disposée dans le canal d'expulsion et/ou le gicleur.

Selon une première variante de réalisation, la substance à action bactériostatique est incorporée au matériau constituant le poussoir et/ou le gicleur.

Selon une seconde variante de réalisation, la substance à action bactériostatique est disposée dans le poussoir et/ou le gicleur sous la forme d'un revêtement mince. Bien entendu, ces deux variantes de réalisation peuvent être combinées pour obtenir une action bactériostatique la plus efficace possible.

Avantageusement, la substance à action bactériostatique comprend des ions d'argent. Ces ions d'argent peuvent notamment être complexés à une matrice minérale.

De préférence, la pompe est une pompe fonctionnant sans reprise d'air.

L'objet de l'invention est de fournir un dispositif de distribution de produit fluide ayant une action oligodynamique. Toutefois, contrairement au dispositif divulgué par le document EP-0 473 892 et à l'opposé de l'enseignement donné par ce document, la pompe de l'invention ne comporte pas de substance à action oligodynamique ou bactériostatique. On évite ainsi un contact permanent entre le produit contenu dans le distributeur et/ou la chambre de pompe et la substance oligodynamique, et la pompe est plus simple et moins coûteuse à fabriquer.

L'invention prévoit au contraire de disposer une substance à action bactériostatique uniquement dans le poussoir, et non pas dans la pompe ou dans le récipient. Bien entendu, une pompe fonctionnant sans reprise d'air, communément appelée "pompe airless" et qui est bien connue dans l'état de la technique, sera préférée du fait qu'elle évite une contamination du produit contenu dans le réservoir après chaque utilisation du distributeur. L'invention ne se limite toutefois pas à ce type de pompe.

En outre, contrairement au dispositif divulgué par le document EP-0 580 460, l'efficacité de l'action bactériostatique est maximale en raison du très petit volume mort restant dans le poussoir. Ceci est vrai aussi bien lors de l'actionnement du distributeur que lors du stockage. En raison du faible volume mort, il n'y a d'une part aucun risque de contamination du produit restant dans le poussoir si le temps de stockage est très court, et d'autre part, il n'y a qu'une très faible quantité de produit qui reste en contact permanent avec la substance bactériostatique, si le temps de stockage est long.

Un avantage particulier de l'invention réside en outre dans le fait qu'elle s'applique à toutes les pompes existantes, en particulier les pompes airless, et qu'il n'est par conséquent pas nécessaire de modifier ou d'adapter lesdites pompes existantes pour obtenir le résultat souhaité. Seul le poussoir est à modifier selon l'invention, d'où il résulte une forte économie de coût dans la fabrication du distributeur de produit.

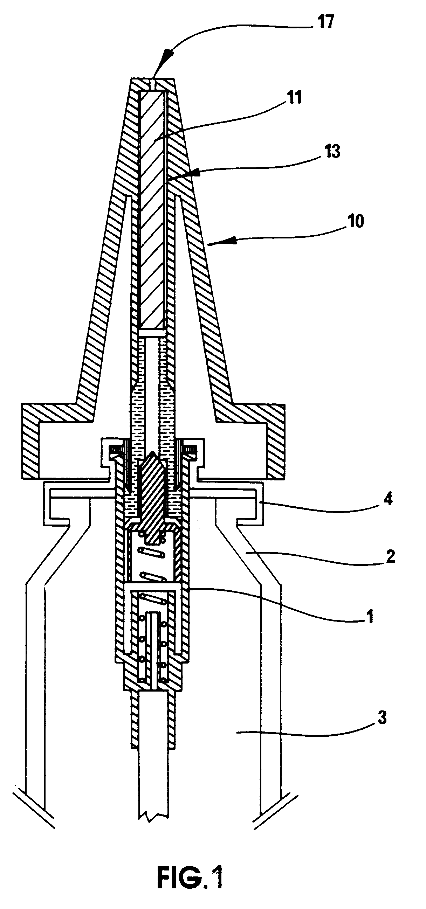

L'invention va maintenant être décrit de manière plus détaillée, à titre d'exemple non limitatif, en regard de la figure 1, qui représente schématiquement en coupe un dispositif de distribution de produit fluide selon l'invention.

En référence à la figure 1, le dispositif de distribution comporte une pompe 1, de préférence une pompe airless, dont le fonctionnement est connu et qui ne sera donc pas décrit plus en détail ci-après. Cette pompe 1 est fixée sur le col 2 d'un récipient 3 d'une manière quelconque, par exemple au moyen d'une bague de fixation 4. Le dispositif comporte en outre un poussoir 10. Ce poussoir 10 comporte un canal d'expulsion 13 reliant la pompe 1 à un orifice de distribution 17 de produit. Selon l'invention, le poussoir 10 comporte un gicleur. Avantageusement, dans l'exemple représenté sur la figure 1, le poussoir 10 est un poussoir nasal et comporte un gicleur interne 11 disposé dans le canal d'expulsion 13, dans lequel débouche directement la pompe 1. Le gicleur interne 11 remplit sensiblement tout le volume disponible entre la pompe 1 et l'orifice de distribution 17, et limite ainsi au maximum le volume mort. Il définit un canal d'expulsion 13 très étroit et de faible volume et favorise avantageusement la pulvérisation du produit. Bien entendu, l'invention s'applique à tout autre type quelconque de poussoir comportant un gicleur.

Selon l'invention, une substance à action bactériostatique est disposée uniquement dans ledit poussoir 10, sur ou à proximité dudit gicleur 11. Ce type de substance bactériostatique est connu dans l'état de la technique et comporte avantageusement des métaux lourds tel que l'argent, notamment sous la forme de sels, par exemple des nitrates ou de chlorures. Une substance adaptée est par exemple constituée d'un alliage de chlorure d'argent et de dioxyde de titane. Une autre substance adaptée est constituée d'ions d'argent complexés à une matrice minérale.

Cette substance anti-bactérienne peut être disposée dans l'une ou plusieurs des parties constitutives du poussoir 10 qui sont en contact avec le produit lors de son expulsion. Ainsi, ladite substance peut être disposée dans le canal d'expulsion 13. Ainsi, le produit qui, lors de son expulsion, traverse le canal d'expulsion 13 vers l'ouverture de sortie 17, vient en contact ponctuel avec la substance bactériostatique et les germes contenus dans le produit sont éliminés. D'autre part, du fait du faible volume mort, seule une très petite quantité de produit restant dans le poussoir entre deux actionnements du distributeur est en contact permanent avec la substance bactériostatique, ce qui évite des problèmes de stabilité du produit.

La substance bactériostatique peut être incorporée dans la matière constituant le poussoir, ou être appliquée sous la forme d'un revêtement mince sur les parties concernées du poussoir 10, à savoir le gicleur 11 et/ou le canal d'expulsion 13.

## Revendications

1. Dispositif de distribution de produit fluide fonctionnant sans reprise d'air comportant une pompe (1) montée sur un récipient (3) contenant le produit, et un poussoir (10) pour actionner ladite pompe, ledit poussoir 10) comprenant un orifice de distribution de produit (17) relié à la pompe (1) par un canal d'expulsion (13), une substance ayant une action bactériostatique sur ledit produit lorsque celui-ci vient en contact avec ladite substance étant disposée dans ledit dispositif de distribution, caractérisé en ce que ledit poussoir (10) comporte un gicleur (11) réalisé sous la forme d'un insert mis en place dans le canal d'expulsion (13) et remplissant sensiblement tout le volume disponible entre la pompe (1) et l'orifice de distribution (17) de manière à limiter au maximum le volume mort, ladite substance à action bactériostatique étant disposée uniquement dans ledit poussoir (10), sur ou à proximité dudit gicleur (11), de sorte que ladite substance à action bactériostatique a une efficacité maximale en raison du faible volume mort dans le poussoir.

2. Dispositif de distribution selon la revendication 1, dans lequel ledit poussoir (10) est un poussoir nasal comportant un gicleur interne (11) remplissant sensiblement tout le volume mort et définissant un étroit canal d'expulsion (13), ladite substance à action bactériostatique étant disposée dans le canal d'expulsion (13) et/ou le gicleur (11).

3. Dispositif de distribution selon la revendication 1 ou la revendication 2, dans lequel la substance à action bactériostatique est incorporée au matériau constituant le poussoir (10) et/ou le gicleur (11).

4. Dispositif de distribution selon l'une quelconque des revendications précédentes, dans lequel la substance à action bactériostatique est disposée dans le poussoir (10) et/ou le gicleur (11) sous la forme d'un revêtement mince.

5. Dispositif de distribution selon l'une quelconque des revendications précédentes, dans lequel la substance à action bactériostatique comprend des ions d'argent.

6. Dispositif de distribution selon l'une quelconque des revendications précédentes, dans lequel la pompe est une pompe fonctionnant sans reprise d'air.

## Claims

1. A device for dispensing a fluid and operating without air intake, the device comprising a pump (1) mounted on a receptacle (3) containing the fluid, and a pushbutton (10) for actuating said pump, said pushbutton (10) including a fluid dispensing orifice (17) connected to the pump (1) via an expulsion channel (13), a substance having bacteriostatic activity on said fluid when it comes into contact with said substance being disposed in said dispenser device, the device being characterized in that said pushbutton (10) includes a nozzle (11) implemented in the form of an insert placed in the expulsion channel (13) and occupying substantially all of the available volume between the pump (1) and the dispensing orifice (17) so as to minimize dead volume, said substance having bacteriostatic activity being disposed solely in said pushbutton (10) on or in the vicinity of said nozzle (11) such that said substance having bacteriostatic activity is of maximum effectiveness because of the small dead volume in the pushbutton.

2. A dispenser device according to claim 1, in which said pushbutton (10) is a nasal pushbutton including an internal nozzle (11) occupying substantially the entire dead volume and defining a narrow expulsion channel (13), said substance having bacteriostatic activity being disposed in the expulsion channel (13) and/or the nozzle (11).

3. A dispenser device according to claim 1 or claim 2, in which the substance having bacteriostatic activity is incorporated in the material constituting the pushbutton (10) and/or the nozzle (11).

4. A dispenser device according to any preceding claim, in which the substance having bacteriostatic activity is disposed in the pushbutton (10) and/or the nozzle (11) in the form of a thin coating.

5. A dispenser device according to any preceding claim, in which the substance having bacteriostatic activity includes ions of silver.

6. A dispenser device according to any preceding claim, in which the pump is a pump that operates without air intake.

## Patentansprüche

1. Abgabevorrichtung für ein Fluid, die ohne Ansaugung von Luft arbeitet und eine auf einem das Produkt enthaltenden Behälter (3) montierte Pumpe (1) und einen Drücker (10) zur Betätigung der Pumpe umfaßt, wobei der Drücker (10) eine Abgabeöffnung (17) für das Produkt aufweist, die mit der Pumpe (1) durch einen Ausstoßkanal (13) verbunden ist, wobei eine Substanz, die auf das Produkt eine bakteriostatische Wirkung ausübt, wenn es mit der Substanz in Berührung kommt, in dieser Abgabevorrichtung vorgesehen ist, dadurch gekennzeichnet, daß der Drücker (10) einen Zerstäuber (11) umfaßt, der in Form eines Einsatzes ausgeführt ist, der in den Ausstoßkanal (13) eingesetzt ist und im wesentlichen das gesamte, zwischen der Pumpe (1) und der Abgabeöffnung (17) zur Verfügung stehende Volumen derart ausfüllt, daß er in maximaler Weise das tote Volumen begrenzt, und daß die Substanz mit bakteriostatischer Wirkung in einzigartiger Weise in dem Drücker (10) an oder in der Nähe des Zerstäubers (11) derart angeordnet ist, daß die Substanz mit bakteriostatischer Wirkung aufgrund des geringen toten Volumens im Drücker eine maximale Wirksamkeit besitzt.

2. Abgabevorrichtung nach Anspruch 1, bei der der Drücker (10) ein zum Einführen in die Nase geeigneter Drücker ist, der einen intemen Zerstäuber (11) umfaßt, der im wesentlichen das gesamte tote Volumen ausfüllt und einen geraden Ausstoßkanal (13) umschließt, wobei die Substanz mit bakteriostatischer Wirkung in dem Ausstoßkanal (13) und/oder dem Zerstäuber (11) angeordnet ist.

3. Abgabevorrichtung nach Anspruch 1 oder 2, bei der die Substanz mit bakteriostatischer Wirkung in dem Material enthalten ist, das den Drücker (10) und/oder den Zerstäuber (11) bildet.

4. Abgabevorrichtung nach einem der vorhergehenden Ansprüche, bei der die Substanz mit bakteriostatischer Wirkung in dem Drücker (10) und/oder dem Zerstäuber (11) in Form einer dünnen Auskleidung vorgesehen ist.

5. Abgabevorrichtung nach einem der vorhergehenden Ansprüche, bei der die Substanz mit bakteriostatischer Wirkung Silberionen umfaßt.

6. Abgabevorrichtung nach einem der vorhergehenden Ansprüche, bei der die Pumpe eine Pumpe ist, die ohne die Ansaugung von Luft arbeitet.
